# FASCICULE DE BREVET EUROPEEN

(11) **EP 4 457 035 B1**
(45) Date de publication et mention de la délivrance du brevet: **01.04.2026**
(21) Numéro de dépôt: 22847590.1
(22) Date de dépôt: 20.12.2022
(51) Int. Cl.: B05B 12/08

(54) **PROCEDE ET DISPOSITIF D'ANALYSE D'UN DISPOSITIF DE PULVERISATION DE PRODUIT FLUIDE PHARMACEUTIQUE**
VERFAHREN UND VORRICHTUNG ZUR ANALYSE EINER VORRICHTUNG ZUM SPRÜHEN EINES PHARMAZEUTISCHEN FLÜSSIGPRODUKTS
METHOD AND DEVICE FOR ANALYSING A DEVICE FOR SPRAYING A PHARMACEUTICAL FLUID PRODUCT

(30) Priorité: 30.12.2021 FR 2114693
(43) Date de publication de la demande: 06.11.2024
(73) Titulaire: APTAR France SAS, 27110 Le Neubourg (FR)
(72) Inventeur: GRANJON, Guillaume, 27400 La Vacherie (FR)
(74) Mandataire: CAPRI
(86) Numéro de dépôt international: PCT/FR2022/052435
(87) Numéro de publication internationale: WO 2023/126598

(56) Documents cités:
- US-A1- 2016 216 108
- US-A1- 2019 376 106

## Description

La présente invention concerne un dispositif et un procédé d'analyse de spray généré par un dispositif de pulvérisation de produit fluide pharmaceutique.

Les dispositifs de pulvérisation de produit fluide pharmaceutique sont bien connus. Ils comportent généralement une tête de pulvérisation pourvue d'un orifice de pulvérisation, assemblée sur un réservoir contenant le produit fluide à distribuer. En particulier dans des applications de pulvérisation nasale, l'efficacité thérapeutique du produit fluide pulvérisé peut dépendre des propriétés du spray généré lors de l'actionnement du dispositif. De manière connue, à la fin de la chaîne de montage, c'est-à-dire lorsque le dispositif de pulvérisation est assemblé, et juste avant d'être expédié chez le fabriquant du produit fluide pharmaceutique pour y être assemblé sur un réservoir correspondant, un certain nombre d'échantillons de dispositifs assemblés sont testés en laboratoire pour vérifier si les propriétés du spray correspondent au cahier des charges prédéfini.

Un inconvénient de ce système est qu'il concerne des dispositifs assemblés, et donc destructeur de ces dispositifs qui ne pourront plus, après avoir été testés, être livrés au client.

De plus, ce système impose une vérification humaine des dispositifs testés, et n'est donc pas totalement automatisable.

Pour surmonter cet inconvénient, le document WO2018130791 propose la visualisation par strioscopie d'un flux d'air comprimé chaud ou froid envoyé à travers une tête de pulvérisation. Cette méthode permet d'évaluer l'angle du spray mais pas sa géométrie ni sa symétrie. Ceci a de plus l'inconvénient d'avoir à prévoir un banc strioscopique, relativement complexe et coûteux, et difficilement adaptable dans une chaine de montage d'un dispositif de pulvérisation de produit fluide, et implique donc soit des tests aléatoires sur une partie seulement des dispositifs fabriqués, soit un ralentissement de la chaine de montage, généralement peu souhaitable.

Les documents EP3047912, JPH0599802 et JPS54127347 décrivent d'autres dispositifs de l'état de la technique.

La présente invention a pour but de surmonter les inconvénients susmentionnés.

En particulier, la présente invention a pour but de fournir un dispositif et un procédé d'analyse d'un dispositif de pulvérisation de produit fluide pharmaceutique qui soit non destructeur des dispositifs testés.

La présente invention a également pour but de fournir un dispositif et un procédé d'analyse qui soit automatisé en grande partie.

La présente invention a aussi pour but de fournir un dispositif et un procédé d'analyse qui permette de tester 100% des dispositifs de pulvérisation, sans ralentir la chaine de montage de manière substantielle.

La présente invention a également pour but de fournir un dispositif et un procédé d'analyse qui soit simple et/ou peu coûteux à fabriquer, à assembler et à utiliser.

La présente a donc pour objet un procédé d'analyse d'un dispositif de pulvérisation de produit fluide pharmaceutique comprenant les étapes suivantes :
- fournir une tête de pulvérisation d'un dispositif de pulvérisation de produit fluide pharmaceutique, ladite tête de pulvérisation comportant un orifice de pulvérisation,
- fournir une surface de réception comportant une plaque perforée comportant une pluralité de trous, chaque trou étant associé à une bille, notamment en polystyrène, guidée dans un canal de guidage, notamment en verre, lesdits canaux de guidage s'étendent parallèlement les uns aux autres jusqu'à une vitre transparente derrière laquelle est disposé une caméra,
- charger ladite plaque perforée avec une charge électrostatique pour attirer par l'électricité statique lesdites billes contre lesdits trous,
- faire passer un flux de gaz à travers ledit orifice de pulvérisation de ladite tête de pulvérisation, et l'envoyer sur ladite surface de réception, lesdites billes contactées par ledit flux de gaz étant déplacées sur ladite vitre,
- visualiser la zone d'impact dudit flux de gaz comprimé sur ladite surface de réception formée par lesdites billes déplacées sur ladite vitre, et
- analyser ladite visualisation de ladite zone d'impact pour déterminer si ladite zone d'impact est conforme ou non conforme à des spécifications prédéterminées.

Avantageusement, ledit flux de gaz est un flux de gaz comprimé.

Avantageusement, ledit flux de gaz est un flux d'air comprimé.

Avantageusement, ladite étape d'analyser comprend de déterminer la géométrie, notamment la symétrie, de la zone d'impact dudit flux de gaz sur ladite surface de réception.

Avantageusement, lesdites spécifications prédéterminées comprennent une étendue planaire prédéterminée de la zone d'impact dudit flux de gaz sur ladite surface de réception, de sorte que les têtes de pulvérisation pour lesquelles ladite étendue planaire est similaire à ladite étendue planaire prédéterminée sont classées conformes, et les têtes de pulvérisation pour lesquelles ladite étendue planaire est différente de ladite étendue planaire prédéterminée sont classés non conformes.

Avantageusement, un cycle d'utilisation comprend les étapes suivantes:
- relier ladite plaque perforée à la terre pour en supprimer toutes charges électriques,
- charger ladite plaque perforée avec une charge électrostatique pour attirer lesdites billes contre lesdits trous,
- générer ledit flux de gaz et l'envoyer à travers ladite tête de pulvérisation sur ladite surface de réception, chaque bille contactée par ledit flux de gaz se détachant de ladite plaque perforée et étant guidée sur ladite vitre,
- visualiser ladite zone d'impact en prenant une image des billes sur ladite vitre au moyen de ladite caméra.

La présente a également pour objet un dispositif d'analyse d'un dispositif de pulvérisation de produit fluide pharmaceutique comprenant :
- une tête de pulvérisation d'un dispositif de pulvérisation de produit fluide pharmaceutique, ladite tête de pulvérisation comportant un orifice de pulvérisation,
- une surface de réception comportant une plaque perforée comportant une pluralité de trous, chaque trou étant associé à une bille, notamment en polystyrène, guidée dans un canal de guidage, notamment en verre, lesdits canaux de guidage s'étendent parallèlement les uns aux autres jusqu'à une vitre transparente,
- un dispositif électrique pour charger ladite plaque perforée avec une charge électrostatique pour attirer lesdites billes par l'électricité statique contre lesdits trous de ladite plaque perforée,
- des moyens de génération d'un flux de gaz pour faire passer un flux de gaz à travers ledit orifice de pulvérisation de ladite tête de pulvérisation et l'envoyer sur ladite surface de réception, lesdites billes contactées par ledit flux de gaz étant déplacées sur ladite vitre,
- une caméra pour visualiser la zone d'impact dudit flux de gaz comprimé sur ladite surface de réception en prenant une image des billes déplacées par ledit flux de gaz sur ladite vitre, et
- des moyens d'analyse pour analyser ladite image de ladite zone d'impact pour déterminer si ladite zone d'impact est conforme ou non conforme à des spécifications prédéterminées.

Avantageusement, ledit flux de gaz est un flux d'air comprimé.

Avantageusement, lesdits trous sont équidistants et proches les uns des autres, formant ainsi un réseau régulier et dense de trous sur ladite surface de réception.

Avantageusement, lesdits moyens de génération du flux de gaz comprimé sont adaptés à générer des impulsions de durée réglable, notamment de 50 à 300 ms.

Ces caractéristiques et avantages et d'autres apparaîtront plus clairement au cours de la description détaillée suivante, faite en référence aux dessins joints, donnés à titre d'exemples non limitatifs, et sur lesquels :
la figure 1 est une vue schématique d'un dispositif pour analyser un dispositif de pulvérisation, selon un mode de réalisation avantageux, avant utilisation,
la figure 2 est une vue schématique similaire à celle de la figure 1, en cours d'utilisation,
la figure 3 montre une visualisation d'une zone d'impact conforme, et
la figure 4 montre une visualisation d'une zone d'impact non-conforme.

Un objectif de l'invention est d'améliorer la qualité du contrôle des dispositifs de pulvérisation. Pour cela, l'invention prévoit d'analyser 100% des dispositifs, sans ralentissement substantiel de la chaine de montage.

De manière classique, chaque dispositif de pulvérisation comprend une tête de pulvérisation 1 pourvue d'un orifice de pulvérisation 2. Généralement, un profil de pulvérisation (non représenté) est prévu en amont dudit orifice de pulvérisation 2 pour générer une forme de spray conique en sortie de l'orifice.

La présente invention prévoit de faire passer un flux de gaz F1, de préférence comprimé, à travers chaque tête de pulvérisation 1, et de diriger ce flux de gaz F1, sortant de l'orifice de pulvérisation 2 avec la forme d'un spray conique, vers une surface de réception 10. Avantageusement, le flux de gaz F1 est un flux d'air comprimé, mais il est entendu que selon l'invention, n'importe quel gaz approprié autre que de l'air pourrait être utilisé.

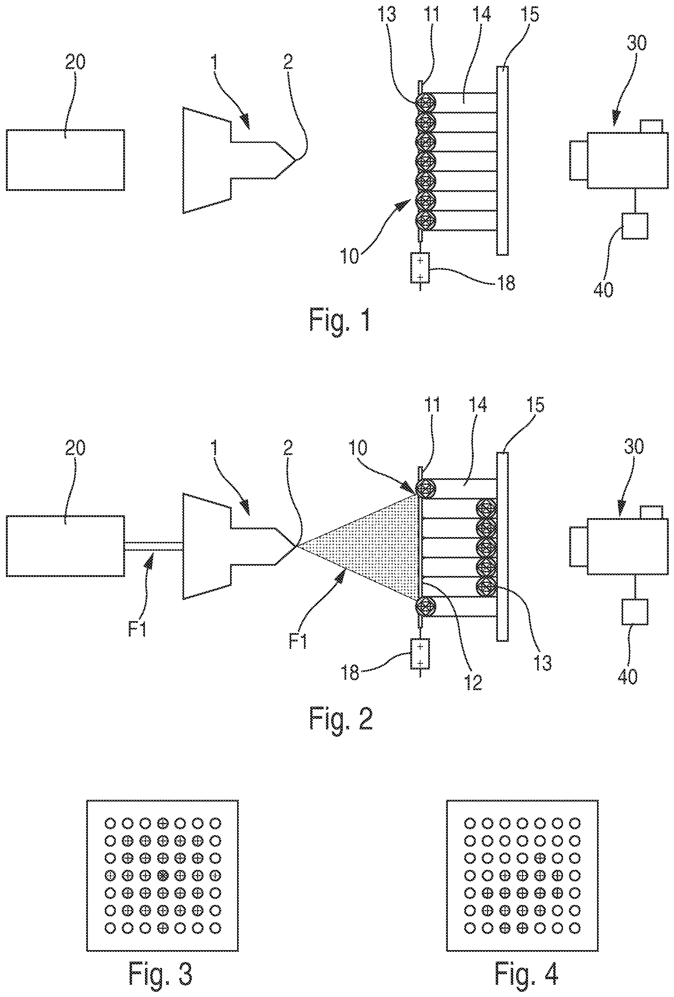

Les figures 1 et 2 montrent un dispositif de test selon un mode de réalisation avantageux.

Dans cet exemple, une tête de pulvérisation 1 est disposée en face d'une surface de réception 10. Des moyens de génération 20 d'un flux de gaz F1 sont prévus pour faire passer un flux de gaz F1 à travers la tête de pulvérisation 1.

La surface de réception 10 forme un plan qui comporte une plaque perforée 11 comportant une pluralité de trous 12. Cette plaque perforée 11 peut être chargée électriquement avec une charge électrostatique. Chaque trou 12 est associé à une bille 13, de préférence en polystyrène, guidée dans un canal de guidage 14, de préférence en verre. Les canaux de guidage 14 s'étendent tous parallèlement les uns aux autres jusqu'à une vitre transparente 15, avantageusement reliée à la terre. Lorsque la plaque perforée 11 n'est pas chargée, les billes 13 sont disposées sur la vitre 15. Lorsque la plaque perforée 11 est chargée, les billes 13 sont attirées par l'électricité statique contre les trous 12 de la plaque perforée 11. Lorsqu'une bille 13 est contactée par le flux de gaz F1, elle se détache de la plaque perforée 11 et est déplacée sur la vitre 15. Ainsi, le motif de billes sur la vitre 15 après l'envoi du flux de gaz F1 sur la surface de réception 10 correspond parfaitement à la zone d'impact dudit flux de gaz F1 sur la surface de réception 10.

Derrière la vitre 15, il est disposé une caméra 30, pour visualiser ce motif de billes et donc la zone d'impact.

Il est à noter que l'orientation du dispositif de test n'est pas nécessairement celle représentée sur les figures 1 et 2, une orientation avantageuse étant verticale, avec le flux de gaz F1 arrivant par le haut sur la plaque perforée 11.

Avant chaque analyse, la plaque perforée 11 est reliée à la terre pour éliminer toutes charges électriques de la surface de réception 10. Ceci fait retomber toutes les billes 13 sur la vitre 15.

Ensuite, la plaque perforée 11 est chargée en appliquant une charge électrostatique au moyen d'un dispositif électrique 18 approprié, ce qui attire chaque bille 13 contre son trou respectif 12 de la plaque perforée 11.

Un flux de gaz F1, notamment d'air comprimé, est alors généré et envoyé à travers la tête de pulvérisation 1 vers la surface de réception 10. La force du flux de gaz F1 décroche les billes 13 de la plaque perforée 11 soumise à une charge électrostatique. En d'autres termes, chaque bille 13 qui est contactée par ledit flux de gaz F1 va se détacher de la plaque perforée 11 et être guidée par son canal de guidage respectif 14 contre la vitre 15.

La caméra 30 prend alors une image de cette vitre 15, avec les billes 13 ayant été impactées par le flux de gaz F1 formant sur ladite vitre 15 la zone d'impact.

Après chaque utilisation, on remet à la masse la plaque perforée 11 et la vitre 15, puis on applique à nouveau une charge électrostatique sur la plaque perforée 11 pour attirer les billes contre les trous 12 de la plaque perforée 11. Le dispositif est alors prêt pour une prochaine utilisation. Eventuellement, on peut contrôler la position des billes 13 avec la caméra 30, et si toutes les billes 13 sont plaquées contre la plaque perforée 11, on peut relancer un nouveau test.

Avantageusement, les trous 12 sont équidistants, et proches les uns des autres, formant ainsi un réseau régulier et dense de trous 12 sur la surface de réception 10. Plus il y a de trous 12 et plus ces trous 12 sont petits, et donc plus il y a de billes 13 et plus ces billes sont petites, plus la définition de la zone d'impact du flux de gaz F1 sur la surface de réception 10 est précise et la forme de cette zone d'impact bien retranscrite.

Pour réaliser les évaluations de conformité, on prévoit une caméra 30 pour visualiser la zone d'impact et des moyens d'analyse 40 pour analyser les visualisations générées par la caméra 30 et ainsi déterminer si la zone d'impact du flux de gaz F1 issu de ladite tête de pulvérisation 1 sur la surface de réception 10 est conforme ou non conforme à des spécifications prédéterminées.

La durée de l'impulsion de gaz F1 est avantageusement réglable, notamment de 50 à 300 ms.

Avantageusement, on peut réaliser plusieurs cycles successifs sur une même tête de pulvérisation, par exemple cinq cycles. La constance ou répétabilité des résultats permet également d'évaluer la conformité de ladite tête de pulvérisation.

Les spécifications prédéterminées peuvent comprendre une étendue planaire prédéterminée de ladite zone d'impact sur ladite surface de réception 10, de sorte que les têtes de pulvérisation 1 pour lesquelles ladite étendue planaire est similaire à ladite étendue planaire prédéterminée sont classées conformes, et les têtes de pulvérisation 1 pour lesquelles ladite étendue planaire est différente de ladite étendue planaire prédéterminée sont classés non conformes. La géométrie, et notamment la symétrie, de la zone d'impact peut aussi être utilisée dans l'évaluation de conformité. D'autres paramètres peuvent aussi être envisagés.

Les moyens d'analyse 40 peuvent comprendre des moyens de mesure de la géométrie de la zone d'impact du flux de gaz F1 sur la surface de réception 10. Par exemple, on détermine le barycentre de la zone d'impact, et on mesure les distances maximale et minimale de ce barycentre du bord de la zone d'impact. La comparaison de ces distances avec des valeurs prédéterminées permet alors d'évaluer la conformité du dispositif testé. Ainsi, l'évaluation de conformité tient compte non seulement de la surface de la zone d'impact, mais aussi de sa géométrie, en particulier sa symétrie. Ceci permet d'établir qu'un spray sortant d'une tête de pulvérisation conforme aura une forme conique acceptable, tant d'un point de vue de l'angle du spray que de sa symétrie.

Eventuellement, des moyens de traitement d'image peuvent être utilisés pour réaliser ce type d'analyse.

Les figures 3 et 4 illustrent chacune une représentation schématique obtenue avec le procédé et le dispositif de l'invention, sur lesquelles il est possible d'évaluer l'étendue planaire et la géométrie, notamment la symétrie, de la zone d'impact. La figure 3 montre une visualisation de la zone d'impact pour un dispositif conforme et la figure 4 montre une telle visualisation pour un dispositif non-conforme.

La présente invention présente de nombreux avantages, et notamment :
- elle permet un contrôle automatisé de conformité sur divers types de dispositif de pulvérisation ;
- elle permet une analyse non destructive desdits dispositifs de pulvérisation ;
- elle permet d'analyser 100% des dispositifs de pulvérisation assemblés sur une chaine de montage, sans ralentissement substantiel de celle-ci ;
- elle permet de réaliser plusieurs tests successifs sur un même dispositif pour évaluer la répétabilité des résultats ;
- elle utilise un montage compact et facilement adaptable ;
- elle utilise des composants simples et standards, donc généralement peu coûteux ;
- elle permet un traitement d'image robuste, qui peut être réalisé en temps réel ;
- elle assure une bonne répétabilité et une bonne discrimination des dispositifs conformes et non-conformes.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Procédé d'analyse d'un dispositif de pulvérisation de produit fluide pharmaceutique, **caractérisé en ce qu'**il comprend les étapes suivantes :
- fournir une tête de pulvérisation (1) d'un dispositif de pulvérisation de produit fluide pharmaceutique, ladite tête de pulvérisation (1) comportant un orifice de pulvérisation (2),
- fournir une surface de réception (10) comportant une plaque perforée (11) comportant une pluralité de trous (12), chaque trou (12) étant associé à une bille (13), notamment en polystyrène, guidée dans un canal de guidage (14), notamment en verre, lesdits canaux de guidage (14) s'étendent parallèlement les uns aux autres jusqu'à une vitre transparente (15) derrière laquelle est disposé une caméra (30),
- charger ladite plaque perforée (11) avec une charge électrostatique pour attirer par l'électricité statique lesdites billes (13) contre lesdits trous (12),
- faire passer un flux de gaz (F1) à travers ledit orifice de pulvérisation (2) de ladite tête de pulvérisation (1), et l'envoyer sur ladite surface de réception (10), lesdites billes (13) contactées par ledit flux de gaz (F1) étant déplacées sur ladite vitre (15),
- visualiser la zone d'impact dudit flux de gaz comprimé (F1) sur ladite surface de réception (10) formée par lesdites billes (13) déplacées sur ladite vitre (15), et
- analyser ladite visualisation de ladite zone d'impact pour déterminer si ladite zone d'impact est conforme ou non conforme à des spécifications prédéterminées.

2. Procédé selon la revendication 1, dans lequel ledit flux de gaz (F1) est un flux de gaz comprimé.

3. Procédé selon la revendication 1 ou 2, dans lequel ledit flux de gaz (F1) est un flux d'air comprimé.

4. Procédé selon l'une quelconque des revendications précédentes, dans lequel ladite étape d'analyser comprend de déterminer la géométrie, notamment la symétrie, de la zone d'impact dudit flux de gaz (F1) sur ladite surface de réception (10).

5. Procédé selon l'une quelconque des revendications précédentes, dans lequel lesdites spécifications prédéterminées comprennent une étendue planaire prédéterminée de la zone d'impact dudit flux de gaz (F1) sur ladite surface de réception (10), de sorte que les têtes de pulvérisation (1) pour lesquelles ladite étendue planaire est similaire à ladite étendue planaire prédéterminée sont classées conformes, et les têtes de pulvérisation (1) pour lesquelles ladite étendue planaire est différente de ladite étendue planaire prédéterminée sont classés non conformes.

6. Procédé selon l'une quelconque des revendications précédentes, dans lequel un cycle d'utilisation comprend les étapes suivantes :
- relier ladite plaque perforée (11) à la terre pour en supprimer toutes charges électriques,
- charger ladite plaque perforée (11) avec une charge électrostatique pour attirer lesdites billes (13) contre lesdits trous (12),
- générer ledit flux de gaz (F1) et l'envoyer à travers ladite tête de pulvérisation (1) sur ladite surface de réception (10), chaque bille (13) contactée par ledit flux de gaz (F1) se détachant de ladite plaque perforée (11) et étant guidée sur ladite vitre (15),
- visualiser ladite zone d'impact en prenant une image des billes (13) sur ladite vitre (15) au moyen de ladite caméra (30).

7. Dispositif d'analyse d'un dispositif de pulvérisation de produit fluide pharmaceutique, **caractérisé en ce qu'**il comprend :
- une tête de pulvérisation (1) d'un dispositif de pulvérisation de produit fluide pharmaceutique, ladite tête de pulvérisation (1) comportant un orifice de pulvérisation (2),
- une surface de réception (10) comportant une plaque perforée (11) comportant une pluralité de trous (12), chaque trou (12) étant associé à une bille (13), notamment en polystyrène, guidée dans un canal de guidage (14), notamment en verre, lesdits canaux de guidage (14) s'étendent parallèlement les uns aux autres jusqu'à une vitre transparente (15),
- un dispositif électrique (18) pour charger ladite plaque perforée (11) avec une charge électrostatique pour attirer lesdites billes (13) par l'électricité statique contre lesdits trous (12) de ladite plaque perforée (11),
- des moyens de génération (20) d'un flux de gaz (F1) pour faire passer un flux de gaz (F1) à travers ledit orifice de pulvérisation (2) de ladite tête de pulvérisation (1) et l'envoyer sur ladite surface de réception (10), lesdites billes (13) contactées par ledit flux de gaz (F1) étant déplacées sur ladite vitre (15),
- une caméra (30) pour visualiser la zone d'impact dudit flux de gaz comprimé (F1) sur ladite surface de réception (10) en prenant une image des billes (13) déplacées par ledit flux de gaz (F1) sur ladite vitre (15), et
- des moyens d'analyse (40) pour analyser ladite image de ladite zone d'impact pour déterminer si ladite zone d'impact est conforme ou non conforme à des spécifications prédéterminées.

8. Dispositif selon la revendication 7, dans lequel ledit flux de gaz (F1) est un flux d'air comprimé.

9. Dispositif selon la revendication 7 ou 8, dans lequel lesdits trous (12) sont équidistants et proches les uns des autres, formant ainsi un réseau régulier et dense de trous (12) sur ladite surface de réception (10).

10. Dispositif selon l'une quelconque des revendications 7 à 9, dans lequel lesdits moyens de génération (20) du flux de gaz comprimé (F1) sont adaptées à générer des impulsions de durée réglable, notamment de 50 à 300 ms.

## Patentansprüche

1. Verfahren zur Analyse einer Sprühvorrichtung für ein pharmazeutisches flüssiges Produkt, **dadurch gekennzeichnet, dass** es die folgenden Schritte aufweist:
- Bereitstellen eines Sprühkopfs (1) einer Sprühvorrichtung für ein pharmazeutisches flüssiges Produkt, wobei der Sprühkopf (1) eine Sprühöffnung (2) aufweist,
- Bereitstellen einer Empfangsfläche (10), die eine perforierte Platte (11) aufweist, die eine Mehrzahl von Löchern (12) aufweist, wobei jedes Loch (12) einer Kugel (13), insbesondere aus Polystyrol, zugeordnet ist, die in einem Führungskanal (14), insbesondere aus Glas, geführt ist, wobei sich die Führungskanäle (14) parallel zueinander bis zu einer transparenten Scheibe (15) erstrecken, hinter der eine Kamera (30) angeordnet ist,
- Aufladen der perforierten Platte (11) mit einer elektrostatischen Ladung, um die Kugeln (13) mittels statischer Elektrizität gegen die Löcher (12) anzuziehen,
- Durchleiten eines Gasstroms (F1) durch die Sprühöffnung (2) des Sprühkopfs (1) und Leiten desselben auf die Empfangsfläche (10), wobei die durch den Gasstrom (F1) beaufschlagten Kugeln (13) auf der Scheibe (15) bewegt werden,
- Visualisieren der Auftreffzone des komprimierten Gasstroms (F1) auf der Empfangsfläche (10), die durch die auf der Scheibe (15) bewegten Kugeln (13) gebildet wird, und
- Analysieren der Visualisierung der Auftreffzone, um zu bestimmen, ob die Auftreffzone vorgegebenen Spezifikationen entspricht oder nicht entspricht.

2. Verfahren nach Anspruch 1, bei dem der Gasstrom (F1) ein komprimierter Gasstrom ist.

3. Verfahren nach Anspruch 1 oder 2, bei dem der Gasstrom (F1) ein komprimierter Luftstrom ist.

4. Verfahren nach einem der vorhergehenden Ansprüche, bei dem der Analyseschritt das Bestimmen der Geometrie, insbesondere der Symmetrie, der Auftreffzone des Gasstroms (F1) auf der Empfangsfläche (10) umfasst.

5. Verfahren nach einem der vorhergehenden Ansprüche, bei dem die vorgegebenen Spezifikationen eine vorgegebene planare Ausdehnung der Auftreffzone des Gasstroms (F1) auf der Empfangsfläche (10) aufweisen, sodass Sprühköpfe (1), bei denen die planare Ausdehnung der vorgegebenen planaren Ausdehnung entspricht, als konform klassifiziert werden und Sprühköpfe (1), bei denen die planare Ausdehnung von der vorgegebenen planaren Ausdehnung abweicht, als nicht konform klassifiziert werden.

6. Verfahren nach einem der vorhergehenden Ansprüche, bei dem ein Nutzungszyklus die folgenden Schritte aufweist:
- Verbinden der perforierten Platte (11) mit Erde, um sämtliche elektrische Ladungen zu entfernen,
- Aufladen der perforierten Platte (11) mit einer elektrostatischen Ladung, um die Kugeln (13) gegen die Löcher (12) anzuziehen,
- Erzeugen des Gasstroms (F1) und Leiten desselben durch den Sprühkopf (1) auf die Empfangsfläche (10), wobei jede durch den Gasstrom (F1) beaufschlagte Kugel (13) sich von der perforierten Platte (11) löst und auf der Scheibe (15) geführt wird,
- Visualisieren der Auftreffzone durch Aufnehmen eines Bildes der Kugeln (13) auf der Scheibe (15) mittels der Kamera (30).

7. Vorrichtung zur Analyse einer Sprühvorrichtung für ein pharmazeutisches flüssiges Produkt, **dadurch gekennzeichnet, dass** sie folgendes aufweist:
- einen Sprühkopf (1) einer Sprühvorrichtung für ein pharmazeutisches flüssiges Produkt, wobei der Sprühkopf (1) eine Sprühöffnung (2) aufweist,
- eine Empfangsfläche (10), die eine perforierte Platte (11) aufweist, die eine Mehrzahl von Löchern (12) aufweist, wobei jedes Loch (12) einer Kugel (13), insbesondere aus Polystyrol, zugeordnet ist, die in einem Führungskanal (14), insbesondere aus Glas, geführt ist, wobei sich die Führungskanäle (14) parallel zueinander bis zu einer transparenten Scheibe (15) erstrecken,
- eine elektrische Vorrichtung (18) zum Aufladen der perforierten Platte (11) mit einer elektrostatischen Ladung, um die Kugeln (13) durch statische Elektrizität gegen die Löcher (12) der perforierten Platte (11) anzuziehen,
- Erzeugungsmittel (20) für einen Gasstrom (F1), um einen Gasstrom (F1) durch die Sprühöffnung (2) des Sprühkopfs (1) zu leiten und auf die Empfangsfläche (10) zu richten, wobei die durch den Gasstrom (F1) beaufschlagten Kugeln (13) auf der Scheibe (15) bewegt werden,
- eine Kamera (30) zum Visualisieren der Auftreffzone des komprimierten Gasstroms (F1) auf der Empfangsfläche (10) durch Aufnehmen eines Bildes der durch den Gasstrom (F1) bewegten Kugeln (13) auf der Scheibe (15), und
- Analysemittel (40) zum Analysieren des Bildes der Auftreffzone, um zu bestimmen, ob die Auftreffzone vorgegebenen Spezifikationen entspricht oder nicht entspricht.

8. Vorrichtung nach Anspruch 7, bei der der Gasstrom (F1) ein komprimierter Luftstrom ist.

9. Vorrichtung nach Anspruch 7 oder 8, bei der die Löcher (12) äquidistant und nahe zueinander angeordnet sind und dadurch ein regelmäßiges und dichtes Lochraster (12) auf der Empfangsfläche (10) bilden.

10. Vorrichtung nach einem der Ansprüche 7 bis 9, bei der die Erzeugungsmittel (20) für den komprimierten Gasstrom (F1) dazu eingerichtet sind, Impulse mit einstellbarer Dauer, insbesondere von 50 bis 300 ms, zu erzeugen.

## Claims

1. A method for analysing a device for spraying a pharmaceutical fluid product, **characterised in that** it comprises the following steps:
- providing a spray head (1) for a device for spraying a pharmaceutical fluid product, said spray head (1) comprising a spray orifice (2),
- providing a receiving surface comprising a perforated plate (11) comprising a plurality of holes (12), each hole (12) being associated with a ball (13), in particular made of polystyrene, guided in a guide channel (14), in particular made of glass, said guide channels (14) extending parallel to one another until reaching a transparent pane (15) behind which a camera is arranged (30),
- charging said perforated plate (11) with an electrostatic charge to attract by static electricity said balls (13) to said holes (12),
- passing a gas stream (F1) through said spray orifice (2) of said spray head (1), and sending it onto said receiving surface (10), said balls (13) that are hit by the gas stream (F1) being moved to said pane (15),
- visualising the impact zone for said compressed gas stream (F1) on said receiving surface (10) that is formed by the balls (13) that have been moved to the pane (15), and
- analysing said visualisation of said impact zone in order to determine whether or not said impact zone complies with predetermined specifications.

2. The method as claimed in claim 1, wherein said gas stream (F1) is a compressed gas stream.

3. The method as claimed in claim 1 or claim 2, wherein said gas stream (F1) is a compressed air stream.

4. The method as claimed in any one of the preceding claims, wherein said step for analysis comprises determining the geometry, in particular the symmetry, of the impact zone for said gas stream (F1) on said receiving surface (10).

5. The method as claimed in any one of the preceding claims, wherein said predetermined specifications comprise a predetermined planar extent of the impact zone for said gas stream (F1) on said receiving surface (10), in a manner such that the spray heads (1) for which said planar extent is similar to said predetermined planar extent are classified as compliant, and the spray heads (1) for which said planar extent is different from said predetermined planar extent are classified as non-compliant.

6. The method as claimed in any one of the preceding claims, wherein an operating cycle comprises the following steps:
- connecting said perforated plate to ground in order to eliminate any electrical charges,
- charging said perforated plate (11) with an electrostatic charge to attract said balls (13) to said holes (12),
- generating said gas stream (F1) and sending it through said spray head (1) onto said receiving surface (10), each ball (13) that is hit by said gas stream (F1) detaching from said perforated plate (11) and being guided onto said pane (15),
- visualising said impact zone by taking an image of the balls (13) on said pane (15) by means of said camera (30).

7. A device for analysing a device for spraying a pharmaceutical fluid product, **characterized in that** it comprises:
- a spray head (1) for a device for spraying a pharmaceutical fluid product, said spray head (1) comprising a spray orifice (2);
- a receiving surface (10) comprising a perforated plate (11) comprising a plurality of holes (12), each hole (12) being associated with a ball (13), in particular made of polystyrene, guided in a guide channel (14), in particular made of glass, said guide channels (14) extending parallel to one another until reaching a transparent pane (15),
- an electrical device (18) for charging said perforated plate (11) with an electrostatic charge to attract said balls (13) by static electricity to said holes (12) of said perforated plate (11),
- means (20) for generating a gas stream (F1) in order to pass a gas stream (F1) through said spray orifice (2) of said spray head (1) and sending it onto said receiving surface (10), said balls (13) that are hit by the gas stream (F1) being moved to said pane (15),
- a camera (30) for visualising the impact zone for said compressed gas stream (F1) on said receiving surface (10) by taking an image of the balls (13) that have been moved by said gas stream (F1) to the pane (15), and
- analysing means (40) for analysing said image of said impact zone in order to determine whether or not said impact zone complies with predetermined specifications.

8. The device as claimed in claim 7, wherein said gas stream (F1) is a compressed air stream.

9. The device as claimed in claim 7 or claim 8, wherein said holes (12) are equidistant from and close to one another, thereby forming a regular and dense array of holes (12) on said receiving surface (10).

10. The device as claimed in any one of claims 7 to 9, wherein said means (50) for generating a compressed gas stream (F1) are adapted to generate pulses of adjustable duration, in particular from 50 to 300 ms.
